# EUROPEAN PATENT APPLICATION

(11) **EP 4 585 234 A1**
(43) Date of publication of application: **16.07.2025**
(21) Application number: 24863816.5
(22) Date of filing: 25.11.2024
(51) Int. Cl.: A61L 27/48, A61L 27/18, A61L 27/20, A61L 27/52, A61L 27/54

(54) **TISSUE REPAIR INJECTION COMPOSITION COMPRISING HYALURONIC ACID GEL FUSED WITH BIODEGRADABLE POLYMER MICROGRANULES USING ECO-FRIENDLY SOLVENT, AND METHOD FOR MANUFACTURING SAME**

(30) Priority: 27.11.2023 KR 20230166477
(71) Applicant: CHO, Eun Ae, Seoul 05764 (KR); PARK, Deok Hoon, Seongnam-si Gyeonggi-do 13555 (KR)
(72) Inventor: CHO, Eun Ae, Seoul 05764 (KR); PARK, Deok Hoon, Seongnam-si Gyeonggi-do 13555 (KR)
(74) Representative: Mewburn Ellis LLP
(86) International application number: PCT/KR2024/018741
(87) International publication number: WO 2025/116455

(57) **Abstract**

The present invention relates to an injection composition for tissue repair treatment including a hyaluronic acid gel fused with biodegradable polymer microgranules prepared using an eco-friendly solvent, and a method of preparing the same. The injection composition for tissue repair treatment according to the present invention has effects that it is harmless to a living organism or the environment because it is prepared using an eco-friendly solvent, and since the biodegradable polymer microgranules have a spherical or nearly spherical shape with an uneven and rough surface, they are not phagocytosed by macrophages but are well adsorbed, thereby effectively inducing collagen production, and since it can be used in the state of a hyaluronic acid gel fused with biodegradable polymer microgranules, it can be used readily without separate dilution or the like, and the effect of tissue repair appears upon injection and for a long time after injection.

## Description

### TECHNICAL FIELD

The present invention relates to an injection composition for tissue repair treatment including a hyaluronic acid gel fused with biodegradable polymer microgranules using an eco-friendly solvent, and a method of preparing the same. More specifically, the present invention relates to an injection composition for tissue repair treatment containing hyaluronic acid fused in a gel state with biodegradable polymer microgranules prepared using an eco-friendly solvent, and a method of preparing the same.

### BACKGROUND ART

Recently, as the issues of aging population and low birth rate deteriorate, the market demand for the elderly-friendly industry and anti-aging industry is rapidly increasing, and the customers of the anti-aging industry for skin treatment, fillers, botulinum toxin, and other beauty care are gradually increasing due to the desire for youth and beauty.

Soft tissue damage may occur on human skin due to external impact, disease, and aging. At this time, wrinkle improvement, contour correction, and tissue repair treatment are carried out by injecting ingredients similar to skin tissue into a specific region to expand soft tissue, materials that are used for this purpose are generally referred to as 'dermal fillers.'

Filler products have been gradually developed in four generations. The first-generation filler is the early cosmetic filler, which is a collagen filler, and it is rarely used at present because it is animal-based. The second-generation hyaluronic acid filler has high biocompatibility and may be removed through a procedure, so it currently accounts for most of the filler products. The third-generation fillers include calcium hydroxyapatite, polymethacrylate (PMMA), and polyacrylamide (PAHG) fillers. They have the advantage in that they are semi-permanent, but since they are not biodegradable, there are difficulties in use due to safety issues. The fourth-generation filler is a biostimulating filler, which is a biodegradable polymer filler that induces long-term skin regeneration by producing collagen and elastin, and its use is now increasing.

Hyaluronic acid fillers are suitable for ameliorating wrinkles and restoring skin volume by injecting cross-linked products of hyaluronic acid into the skin to supply moisture and recover elasticity. The structure of hyaluronic acid, the raw material, is composed of a polysaccharide backbone in which dimers of N-acetyl-D-glucosamine and D-glucuronic acid are repeated, and it is a component capable of containing water up to 1,000 times its weight through hydrogen bonds. On the other hand, since it has high biodegradability (24 to 48 hours), filler products are prepared by increasing the half-life of hyaluronic acid in the body through cross-linking, and divinyl sulfone (DVS), 1,4-butanediol diglycidyl ether (BDDE), ethylene glycol diglycidyl ether (EGDGE), and the like are used as cross-linking agents. Even after crosslinking, hyaluronic acid is decomposed very quickly in the body by decomposing enzymes and reactive oxygen species (ROS), so the effect of tissue repair treatment is limited to about 3 to 12 months. There are disadvantages in that in order to maintain the effect, reinjection is usually required every several months, and regular management is required. Attempts to develop effective crosslinking technologies are being made such as varying the crosslinking agent and increasing the degree of crosslinking in order to overcome the disadvantages (e.g., Korean Patent Publication No. 10-2022-0099922), but a more active alternative to overcome the inherent limitations of hyaluronic acid is required at present.

Meanwhile, polylactic acid or the like, a biocompatible biodegradable polymer, has been used as a fourth-generation semi-permanent filler material. It is a substance that has a mechanism of gradually promoting collagen synthesis by stimulating macrophages while being biodegraded over time after skin injection, thereby improving the overall skin conditions and exhibiting anti-aging effects. Although it has the advantage that the effects last two to three years due to its slow absorption rate in the body after injection, it has the limitation that no immediate effect is exhibited after the procedure when used for tissue repair treatment. To overcome this limitation, the practitioner needs to provide sufficient explanation about this before the procedure, and insufficient explanation often leads to immediate claims about the efficacy. Furthermore, due to the nature of the polymer, it is difficult to homogeneously disperse the polymer from an aqueous phase to an injection, which causes aggregation, causing the injection needle to be clogged or making injection difficult. To improve this, products that are complexed with carboxymethyl cellulose or non-crosslinked hyaluronic acid have been commercialized, but since they are provided in a powder form, they may only be used after adding an injection agent or buffer solution before the procedure and shaking the mixture continuously or intermittently for a long time to disperse them. In addition, unlike hyaluronic acid fillers, no immediate improvement effect is exhibited after injection, and problems such as volume loss and nodule formation often occur within one month after injection.

Since biodegradable polymers may not be completely dissolved in an aqueous solution on their own, when the suspension process prior to use is not perfect, the injection volume may not be controlled due to needle clogging or unstable injection force, and problems such as crystallization may occur in the region where a large amount is injected. To improve this, attempts are made to provide a small device for automatic stirring or to make the shape of the active ingredient round to increase the surface area and facilitate dispersion. In addition, because of the need for suspension time, there is the inconvenience that customers to receive treatment must visit the clinic right at the scheduled time. When they arrive too early, they have to wait, and even when they arrive on time, when the product they want to be treated with is not well suspended, they have to wait, which creates an inconvenient situation not only for the person providing the treatment but also for the customer receiving the treatment.

In addition, biodegradable polymers are required to be prepared as microparticles for *in vivo* injection, but conventional methods have been based on dissolution mainly in halogenated organic solvents such as methylene chloride and chloroform. According to the CHEM21 selection guide, these organic solvents are classified as "hazardous" or "highly hazardous" solvents, and have toxicity problems and environmental issues (Green Chem, 2016, 18, 288-296). Furthermore, additional facilities for the safety of workers, such as explosion-proof and hood systems and organic solvent dust collection systems, are essential for production facilities, so the production costs are high, and there are difficulties in always having to pay attention to the safety of workers. In addition, conventional biodegradable polymer microparticles have a smooth surface that is not easily adsorbed by macrophages, which causes a negative effect on the mechanism leading to further collagen production stimulation. At this point, when biodegradable polymers may be prepared as microparticles with a rough surface structure using an eco-friendly solvent, it will have great social and economic ripple effects.

Accordingly, the present inventors have attempted to develop a new type of filler that does not require a suspension process prior to use and is already homogeneously dispersed for immediate use, by solving the problems of the conventional biodegradable polymer-containing filler products, such as the need for a long-time suspension process prior to use and the need for practicing caution for homogeneous dispersion during suspension. In addition, the present inventors have attempted to develop a new type of filler that resolves the harmfulness to the human body and the environment caused by the use of organic solvents that are harmful to the human body during the preparation process, and at the same time, includes biodegradable polymer microparticles with a rough surface structure to smoothly stimulate collagen production by the biodegradable polymer.

### DETAILED DESCRIPTION OF THE INVENTION

### TECHNICAL PROBLEM

Therefore, one object of the present invention is to provide an injection composition for tissue repair treatment including hyaluronic acid gel fused with biodegradable polymer microgranules, the injection composition having particle homogeneity and being ready for use as it is already homogeneously dispersed and does not require a long-time suspension process prior to use.

In addition, another object of the present invention is to provide an injection composition for tissue repair treatment including hyaluronic acid gel fused with biodegradable polymer microgranules, the injection composition being eco-friendly and having reduced human and environmental harmfulness as an eco-friendly solvent is used.

In addition, still another object of the present invention is to provide an injection composition for tissue repair treatment including hyaluronic acid gel fused with biodegradable polymer microgranules that are not smooth but have a rough surface structure.

In addition, yet another object of the present invention is to provide a method of preparing an injection composition for skin repair including hyaluronic acid gel fused with the biodegradable polymer microgranules.

### TECHNICAL SOLUTION

As one aspect, the present invention provides a method of preparing an injection composition for tissue repair treatment including a hyaluronic acid gel fused with biodegradable polymer microgranules.

As a specific aspect, the method of preparing an injection composition for tissue repair treatment includes:
(S10) a step of preparing microgranules of a biodegradable polymer using an eco-friendly solvent;
(S20) a step of preparing a hyaluronic acid gel; and
(S30) a step of dispersing the biodegradable polymer microgranules of Step (S10) in the hyaluronic acid gel of Step (S20).

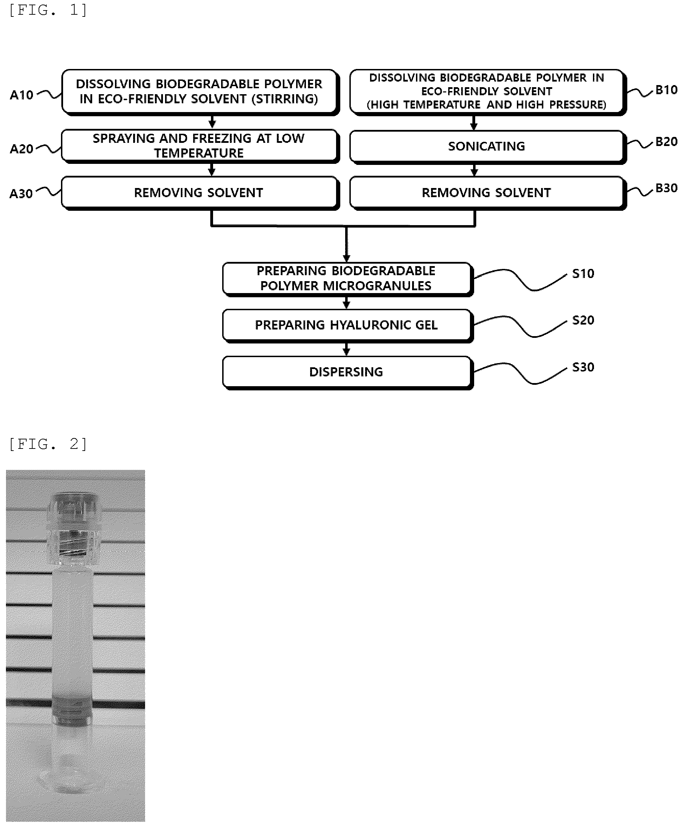

Hereinafter, referring to FIG. 1, a method of preparing an injection composition for tissue repair treatment including a hyaluronic acid gel fused with biodegradable polymer microgranules of the present invention will be specifically described.

First, Step (S10) is a step of preparing microgranules of a biodegradable polymer using an eco-friendly solvent.

In the present invention, the biodegradable polymers may be one or more selected from the group consisting of poly-L-lactic acid (PLLA), poly-D-lactic acid (PDLA), poly-DL-lactic acid (PDLLA), poly-lactic acid (PLA), polyglycolic acid (PGA), polydioxanone (PDO), polycaprolactone (PCL), poly-α-hydroxy acid (PHA), poly(trimethylene carbonate), poly(alkyl cyanoacrylate), polyhydroxyalkanoates, methoxypolyethylene glycol, hydroxypolyethylene glycol (hydroxyl PEG), monoalkoxy polyethylene glycol, polyethylene glycol (PEG), a copolymer thereof, and a mixture thereof. Examples of the copolymer thereof may include a poly(lactic acid)-glycolic acid copolymer, a poly(dioxanone)-caprolactone copolymer, a poly(lactic acid)-caprolactone copolymer, and the like.

As a specific example, the biodegradable polymer may be any one of PLA, PLLA, PDLA, PDLLA, PCL, or the like, and since these are decomposed in water, carbon dioxide, and sugars and absorbed into the body, they are biostable polymer materials.

The biodegradable polymer included in the present invention may have a weight average molecular weight of 10,000 to 500,000 g/mol, preferably 10,000 to 400,000 g/mol, and more preferably 20,000 to 300,000 g/mol. When the molecular weight is less than 10,000, the biodegradable polymer is difficult to have persistence in the body and promote collagen production, and when the molecular weight is more than 500,000, the biodegradable polymer is difficult to process and thus is not suitable for producing desired microparticles.

In the present invention, the eco-friendly solvent may be an eco-friendly organic solvent that has been approved for use in pharmaceutical formulations in the United States and other countries and corresponds to a Class 3 solvent, which is the safest category with low toxicity potential, or is known to have low toxicity potential, triacetin, or a eutectic solvent.

The Class 3 organic solvent may be one or more selected from the group consisting of dimethyl sulfoxide, acetic acid, acetone, anisole, 1-butanol, 2-butanol, butyl acetate, tert-butyl methyl ether, ethanol, ethyl acetate, ethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, and a mixture thereof.

The eco-friendly solvent other than the above-mentioned Class 3 organic solvents may be one or more selected from the group consisting of dihydrolevoglucosensone, methyl-5-(dimethylamino)-2-methyl-5-oxopentanoate, triethyl phosphate, organic carbonates (e.g., ethylene carbonate, propylene carbonate, butylene carbonate, glycerol carbonate, diethyl carbonate, dimethyl carbonate, etc.), lactate esters (e.g., methyl lactate, ethyl lactate, etc.), tetraglycol, and a mixture thereof.

As one specific example, the eco-friendly solvent may be one or more selected from the group consisting of a Class 3 organic solvent, an eco-friendly organic solvent other than a Class 3 organic solvent, and a mixture thereof.

As one specific example, the eco-friendly organic solvent may be dimethyl sulfoxide. The dimethyl sulfoxide is an eco-friendly organic solvent that belongs to the Class 3 solvent, which is the safest category with low toxicity potential.

The triacetin was approved as a food additive generally recognized as safe (GRAS) by the FDA in 1975, and is a safe eco-friendly solvent included in the Select Committee on GRAS substances (SCOGS) database.

The eutectic solvent is a mixture of two or more components mixed by hydrogen bonding and having a lower melting point than each component. The two or more components serve as hydrogen bond acceptors and hydrogen bond donors for hydrogen bonding, and since they have a lower melting point than each component, the eutectic solvent is present as a transparent liquid, and a hydrogen bond network is formed therein, and thus the solvent is an eco-friendly solvent with high dissolving ability.

The hydrogen bond acceptor of the eutectic solvent may include one or more selected from the group consisting of choline chloride, betaine, sodium acetate, proline, nicotinamide, L-carnitine, and a combination thereof.

The hydrogen bond donor of the eutectic solvent may include one or more selected from the group consisting of polyols, organic acids, fatty acids, amide compounds, sugars, and a combination thereof.

The polyol may include, for example, one or more selected from the group consisting of glycerol, erythritol, mannitol, sorbitol, xylitol, lactitol, ethylene glycol, propylene glycol, ribitol, aldonicol, butylene glycol, pentylene glycol, and hexylene glycol.

The sugar may include, for example, one or more selected from the group consisting of glucose, fructose, xylose, arabinose, sucrose, lactose, and galactose.

The organic acid may include, for example, one or more selected from the group consisting of lactic acid, formic acid, acetic acid, propionic acid, malic acid, maleic acid, pyruvic acid, fumaric acid, succinic acid, citric acid, acetic acid, aconitic acid, hyaluronic acid, hydroxycitric acid, levulinic acid, and lactone derivatives.

The fatty acid may include, for example, one or more selected from the group consisting of octanoic acid, nonanoic acid, undecylic acid, capric acid, lauric acid, palmitic acid, linolenic acid, and γ (gamma)-linolenic acid.

The amide compound may include one or more selected from the group consisting of urea, thiourea, acrylamide, acetamide, ceramide, nylon-6, melatonin, and asparagine.

The hydrogen bond donors such as chloride choline, betaine, and proline include those selected from amides, sugars, alcohols, organic acids, fatty acids, and the like.

As a specific example, the eutectic solvent may be used by mixing betaine as a hydrogen bond acceptor and glycerin as a hydrogen bond donor with water.

In the present invention, the biodegradable polymer microgranules are biodegradable polymer particles having a diameter of 20 µm or more that are not phagocytosed by macrophages. When the diameter is less than 20 µm, side effects such as granulomas or inflammatory reactions may occur, and thus microgranules having the diameter are unsuitable (J Drugs Dermatol., 2014, 13, s29-31). In addition, the smaller the diameter of the injection needle, the more effective injection and precise manipulation of the injection solution are possible, and there is the advantage that the flow resistance of the fluid is small, and thus skin irritation is less. Accordingly, the biodegradable polymer microgranules should be smaller than the diameter of the syringe needle in order to enable injection using the syringe needle according to the purpose of the present invention, and so the microgranules preferably have a diameter of 100 µm or less in consideration of the diameter of the smallest syringe needle. As a specific example, the biodegradable polymer microgranules according to the present invention have a diameter of 20 to 100 µm.

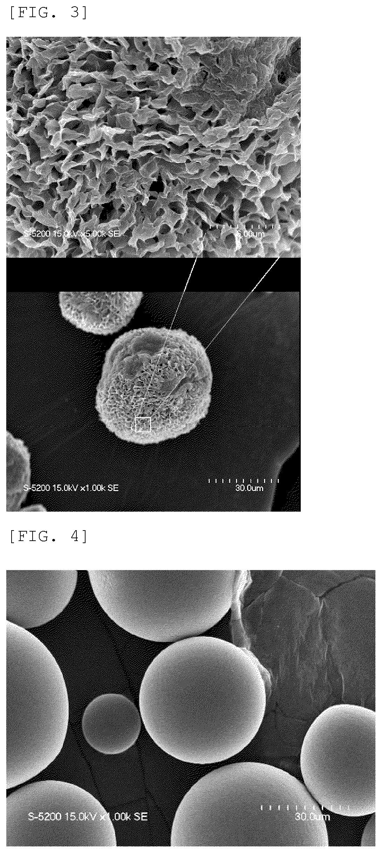

In the present invention, the biodegradable polymer microgranules have a spherical or nearly spherical shape with an uneven and rough surface (see FIG. 3). Here, the nearly spherical shape means that the sphericity, i.e., the ratio of thickness to diameter is 1:0.7 to 1:1.3, preferably 1:0.8 to 1:1.2. When the biodegradable polymer microgranules have an uneven and rough surface, adsorption of macrophages can be achieved well to promote collagen production by fibroblasts and the like through the stimulation of macrophages.

In the present invention, preparation of biodegradable polymer microgranules prepared using the eco-friendly solvent may consist of: (A10) a step of mixing a biodegradable polymer with an eco-friendly solvent and stirring the resulting mixture and dissolving the biodegradable polymer to prepare a biodegradable polymer solution; (A20) a step of spraying and freezing the biodegradable polymer solution of Step (A10) at a low temperature; and (A30) a step of removing the solvent from the frozen biodegradable polymer solution of Step (A20).

Step (A10) is a step of mixing a biodegradable polymer with an eco-friendly solvent and stirring and dissolving to prepare a biodegradable polymer solution, and an eco-friendly solvent may be mixed and dissolved in an amount of 200 to 10,000 parts by weight, preferably 300 to 5,000 parts by weight, and more preferably 500 to 1,000 parts by weight, based on 100 parts by weight of the biodegradable polymer. At this time, as the eco-friendly solvent, the above-described organic solvent, triacetin, or eutectic solvent may be used.

Step (A20) is a step of spraying and freezing the biodegradable polymer solution of Step (A10) at a low temperature, and the low temperature may be a temperature below the freezing point of the solvent and below the freezing point of the biodegradable polymer and may vary depending on the types of the solvent and the biodegradable polymer, but for example, -25 °C to -5 °C, preferably -20 °C to -8 °C, may be suitable.

The spraying refers to applying pressure to a biodegradable polymer solution to spray it with a diameter of less than 100 µm, and the sprayed biodegradable polymer solution is present in the form of frozen solid aerosols due to the above-described low ambient temperature during the spraying.

Step (A30) is a step of removing the solvent from the frozen biodegradable polymer solution of Step (A20), and the removing of the solvent may be achieved by methods such as adsorption, evaporation, and washing, depending on the types of solvent used.

Meanwhile, the biodegradable polymer microgranules prepared by removing the solvent may be dried to facilitate the subsequent dispersion process with a hyaluronic acid gel, and the drying may be achieved by freeze drying, spray drying, vacuum drying, or the like.

In the present invention, preparation of biodegradable polymer microgranules prepared using the eco-friendly solvent may consist of: (B10) a step of mixing a biodegradable polymer and an eco-friendly solvent and dissolving the biodegradable polymer at a high temperature and a high pressure to prepare a biodegradable polymer solution; (B20) a step of sonicating a resulting melt of the biodegradable polymer of Step (B10); and (B30) a step of removing the solvent from the melt of Step (B20).

Step (B10) is a step of mixing a biodegradable polymer and an eco-friendly solvent and dissolving the biodegradable polymer at a high temperature and a high pressure to prepare a biodegradable polymer solution, and an eco-friendly solvent may be mixed and dissolved in an amount of 200 to 10,000 parts by weight, preferably 300 to 5,000 parts by weight, and more preferably 500 to 1,000 parts by weight, based on 100 parts by weight of the biodegradable polymer. At this time, as the eco-friendly solvent, the above-described organic solvent, triacetin, or eutectic solvent may be used.

In the high temperature and the high pressure, the high temperature may be 60 °C or higher, 60 to 200 °C, 70 to 190 °C, 120 to 180 °C, 80 to 180 °C, 90 to 180 °C, 100 to 180 °C, 120 to 170 °C, 140 to 170 °C, or 160 to 170 °C, and the high pressure may be 0.11 to 20 MPa, 0.11 to 10 MPa, 0.11 to 5 MPa, 0.11 to 2 MPa, 0.11 to 1 MPa, 0.11 to 0.5 MPa, 0.11 to 0.3 MPa, 0.11 to 0.2 MPa, or 0.12 MPa. In addition, the time for the treatment at the high temperature and the high pressure is sufficient as long as the biodegradable polymer is sufficiently dissolved in the eco-friendly solvent, and is preferably 10 to 30 minutes, more preferably 15 to 25 minutes, and even more preferably 18 to 22 minutes.

Step (B20) is a step of sonicating the biodegradable polymer melt of Step (B10), and the sonication suppresses further agglomeration of the biodegradable polymer microgranules. The sonication may be performed for 10 to 60 minutes, preferably 20 to 30 minutes.

Step (B30) is a step of removing the solvent from the melt of Step (B20), and the removing of the solvent may be achieved by methods such as adsorption, evaporation, and washing, depending on the types of solvent used.

Meanwhile, the biodegradable polymer microgranules prepared by removing the solvent may be dried to facilitate the subsequent dispersion process with a hyaluronic acid gel, and the drying may be achieved by freeze drying, spray drying, vacuum drying, or the like.

Step (S20) is a step of preparing a hyaluronic acid gel.

In the present invention, the hyaluronic acid gel refers to a hydrogel in a hydrated state in which a cross-linking agent is added to a hyaluronic acid aqueous solution to cross-link hyaluronic acid.

In the present invention, the term 'hyaluronic acid' is used to refer to not only hyaluronic acid itself but also salts and derivatives thereof. Therefore, the term 'hyaluronic acid aqueous solution' used herein is a concept incorporating an aqueous solution of hyaluronic acid, an aqueous solution of a hyaluronic acid salt, an aqueous solution of a hyaluronic acid derivative, and an aqueous solution of a mixture thereof.

The hyaluronic acid may have an average molecular weight of, for example, 50,000, 60,000, 100,000, 200,000, 500,000, 800,000, 1,000,000, 1,200,000, 1,500,000, 1,800,000, 2,000,000, 2,200,000, 2,500,000, 2,800,000, 2,900,000, and 3,000,000. The average molecular weight may be within the range of any two values exemplified herein. Considering the crosslinked gel of hyaluronic acid, the average molecular weight is preferably 1,000,000 or more, and preferably 1,500,000 or more.

The hyaluronic acid aqueous solution is prepared by dissolving hyaluronic acid in an alkaline solution, and the alkaline solution has a pH of 11 to 13, preferably a pH of 11.5 to 12.5, and examples thereof include a sodium hydroxide solution, a magnesium hydroxide solution, a calcium hydroxide solution, and the like.

As the crosslinking agent, a substance having excellent biocompatibility and capable of crosslinking hyaluronic acid may preferably be used, and examples thereof include compounds such as 1,4-butanediol diglycidyl ether (BDDE), ethylene glycol diglycidyl ether (EGDGE), divinyl sulfone (DVS), polyethylene glycol (PEG), polyethylene glycol diglycidyl ether, bis(ethylcarbodiimide) (BCDI), 1,6-hexanediol diglycidyl ether, N-hydroxysuccinimide (NHS), 1-ethyl-3(3-dimethylaminopropyl)carbodiimide (EDC), hexamethylenediamine (HMDA), 1,2,7,8-diepoxyoctane (DEO), citric acid, propylene glycol diglycidyl ether, polypropylene glycol diglycidyl ether, polytetramethylene glycol diglycidyl ether, neopentyl glycol diglycidyl ether, polyglycerol polyglycidyl ether, diglycerol polyglycidyl ether, glycerol polyglycidyl ether, trimethylpropane polyglycidyl ether, 1,2-bis(2,3-epoxypropoxy)ethylene, pentaerythritol polyglycidyl ether, and sorbitol polyglycidyl ether. Preferably, BDDE, EGDGE, DVS, BCEDI, and the like may be used.

As a crosslinking method of hyaluronic acid using the crosslinking agent, all known technologies may be used, and the present invention is not limited to the crosslinking method. As a specific example, preparation of the hyaluronic acid gel may be achieved by dissolving hyaluronic acid in a sodium hydroxide solution of pH 12, adding a crosslinking agent, mixing, and then maturing the resulting mixture at room temperature under nitrogen conditions.

Meanwhile, after the preparation of the hyaluronic acid gel, the crosslinking agent is preferably removed as much as possible for biostability, and the removal of the crosslinking agent may be achieved after the preparation of the hyaluronic acid gel in Step (S20) and before Step (S30) described below, or after Step (S30) described below. At this time, the removal of the crosslinking agent may be carried out using various methods known in the art, such as those disclosed in Korean Patent No. 10-1922711 and Korean Patent No. 10-1044339, and a specific example includes a dialysis method.

Step (S30) is a step of dispersing the biodegradable polymer microgranules of Step (S10) in the hyaluronic acid gel of Step (S20).

The hyaluronic acid gel of Step (S20) used in Step (S30) may be a hydrated hybrid gel prepared in Step (S20) and may be a hyaluronic acid gel from which the crosslinking agent is either removed or not, as described above.

The dispersion may prepared by stirring hyaluronic acid and biodegradable polymer microgranules in a weight ratio of 1:0.5 to 5, preferably 1:0.5 to 4, more preferably 1:0.5 to 3, and even more preferably 1:0.5 to 2.5. When the weight ratio of the biodegradable polymer microgranules to the hyaluronic acid is less than 0.5, there is a problem that the tissue repair ability of the finally prepared composition for repair treatment is excellent at the initial stage of injection, but the repair ability decreases over time, and there is also a problem that continuous tissue repair treatment due to the expected collagen production ability is insufficient compared to existing hyaluronic acid fillers. In addition, when the weight ratio of the biodegradable polymer microgranules to the hyaluronic acid exceeds 5, a stable hyaluronic acid fusion gel may not be formed due to the excessive amount of the biodegradable polymer, and biologically, there is a possibility of causing inflammation.

In addition, in order to adjust the amount of the hyaluronic acid gel and biodegradable polymer microgranules contained in the finally prepared composition during the dispersion process or to impart additional uses, one or more selected from the group consisting of purified water, a physiologically active substance, and a local anesthetic may be further included.

The amount of hyaluronic acid contained in the finally prepared composition may be 1% to 5% by weight based on the percentage by weight of the total composition. When the amount is less than 1% by weight, the effect of tissue repair treatment is minimal. When the amount is more than 5% by weight, the hyaluronic acid gel is very hard, and therefore fails to have an injection force that enables injection with a syringe. In addition, the amount of biodegradable polymer microgranules contained in the finally prepared composition may be 1% to 10% by weight based on the percentage by weight of the total composition. When the amount is less than 1% by weight, the effect of tissue repair treatment is minimal. When the amount is more than 10% by weight, the acidity increases so that the pH of the composition becomes less than 6.0, making the composition unsuitable for tissue repair treatment.

The physiologically active substance may be a component useful for biocompatibility and skin tissue repair treatment, and examples thereof include mannitol, inositol, allantoin, matrine, phytic acid, niacinamide, vitamin C and derivatives thereof, ectoine, ergothioneine, glutathione, nicotinamide, nicotinamide riboside, nicotinamide mononucleotides, peptides, amino acids, carnosine, exosomes, and the like, but are not necessarily limited thereto. The physiologically active substance may be included in an amount of 0.01% to 3.0% by weight, preferably 0.05% to 2.5% by weight, and more preferably 0.05% to 2.0% by weight based on the percentage by weight of the total composition. When the amount of the physiologically active substance is less than 0.01% by weight, the physiologically active effect due to the addition of the physiologically active substance may be minimal, and when the amount of the physiologically active substance exceeds 3.0% by weight, there is a problem that the hyaluronic acid gel fused with the biodegradable polymer microgranules of the composition may be decomposed by the physiologically active substance, which is not preferable.

The local anesthetic may include lidocaine, mepivacaine, levobupivacaine, and prilocaine, and components such as local anesthetics may be included. The amount of the local anesthetic may be appropriately adjusted by a person skilled in the art, but it may preferably be contained in an amount of 0.1% to 0.5% by weight based on the percentage by weight of the total composition.

The hyaluronic acid gel fused with the biodegradable polymer microgranules prepared by the above-described method is a turbid gel that maintains its dispersed state as it is without being separated over time and, and since the pH is neutral at 6.5 to 7.5, there is no problem in administering it to skin tissue.

Therefore, the prepared hyaluronic acid gel fused with the biodegradable polymer microgranules may be provided as a composition for tissue repair treatment, and the composition may be used as it is, but preferably, it can be used after being packed in a pre-filled syringe and then sealed.

As a specific example, the prepared hyaluronic acid gel fused with the biodegradable polymer microgranules may be injected into skin tissue at a uniform pressure at an injection pressure of 40 N or less after being packed in a pre-filled syringe.

Therefore, the hyaluronic acid gel fused with the biodegradable polymer microgranules prepared by the above-described method has a syringe pressure of 40 N or less when measured at an injection speed of 5 to 15 mm/min with a syringe of 27G (I.D. 0.210 mm).

As another aspect, the present invention provides an injection composition for tissue repair treatment including a hyaluronic acid gel fused with biodegradable polymer microgranules.

In the present invention, tissue repair treatment may mean temporarily or semi-permanently ameliorating or recovering wrinkles on the face or body, or regenerating tissues such as contour improvement, tissue volume formation, or scar healing by injecting the composition. The tissue may mean a part of the face or body.

The injection composition for tissue repair treatment may be filled into a syringe, preferably a pre-filled syringe, and injected into the skin layer. In addition, unless specifically mentioned, the composition for tissue repair treatment in the present specification may also be described as a filler or dermal filler.

In addition, since the composition for tissue repair treatment includes a hyaluronic acid gel fused with biodegradable polymer microgranules, not only does the effect of tissue repair treatment appear immediately after the composition is injected into the tissue, but the effect of tissue repair treatment by the biodegradable polymer microgranules is maintained even after a long period of time, thereby increasing satisfaction with tissue repair treatment.

In addition, since the composition for tissue repair treatment includes a hyaluronic acid gel fused with biodegradable polymer microgranules, the gel has a syringe pressure of 40 N or less when measured at an injection speed of 5 to 15 mm/min with a syringe of 27G (I.D. 0.210 mm), so that the injection of the composition to the tissue can be easy, and the pain of the person receiving the injection can be reduced.

In the injection composition for skin repair treatment of the present invention, the biodegradable polymer microgranules refer to biodegradable polymer particles having a spherical or nearly spherical shape with a rough surface and a diameter of 20 µm to 100 µm, which are prepared by using an eco-friendly solvent.

These biodegradable polymer microgranules may be prepared by the method described in the above-described preparation method, and since they are prepared using an eco-friendly solvent, biodegradable polymer microgranules with reduced harmfulness to the human body and the environment can be provided.

The amount of the biodegradable polymer microgranules may be 1% to 10% by weight based on the percentage by weight of the total composition, and when the amount is less than 1% by weight, the effect of tissue repair treatment is minimal, and when it exceeds 10% by weight, the acidity increases so that the pH of the composition becomes less than 6.0, making the composition unsuitable for tissue repair treatment.

In the composition for skin repair treatment of the present invention, the hyaluronic acid gel fused with the biodegradable polymer microgranules may be prepared by the method described in the above-described preparation method, and the hyaluronic acid, crosslinking agent, and the like are as described above.

The amount of the hyaluronic acid may be 1% to 5% by weight based on the percentage by weight of the total composition. When the content is less than 1% by weight, the effect of tissue repair is minimal. When it exceeds 5% by weight, that the hyaluronic acid gel is too hard, which causes a problem in that it may not be injected with a syringe.

The hyaluronic acid gel fused with the biodegradable polymer microgranules may consist of hyaluronic acid and biodegradable polymer microgranules in a weight ratio of 1:0.5 to 5, preferably 1:0.5 to 4, more preferably 1:0.5 to 3, and even more preferably 1:0.5 to 2.5. When the weight ratio of the biodegradable polymer microgranules to the hyaluronic acid is less than 0.5, there is a problem that the tissue repair ability of the finally prepared composition for repair treatment is excellent at the initial stage of injection, but the repair ability decreases over time, and there is also a problem that continuous tissue repair treatment due to the expected collagen production ability is insufficient compared to existing hyaluronic acid fillers. In addition, when the weight ratio of the biodegradable polymer microgranules to the hyaluronic acid exceeds 5, a stable hyaluronic acid fusion gel may not be formed due to the excessive amount of the biodegradable polymer, and biologically, there is a possibility of causing inflammation.

In the injection composition for skin repair treatment of the present invention, in order to adjust the amount of the hyaluronic acid gel and biodegradable polymer microgranules to the above-described amount, or to impart additional uses, one or more selected from the group consisting of purified water, a physiologically active substance, and a local anesthetic may be further included.

The physiologically active substance may be a component useful for biocompatibility and skin tissue repair treatment, and examples thereof include mannitol, inositol, allantoin, matrine, phytic acid, niacinamide, vitamin C and derivatives thereof, ectoine, ergothioneine, glutathione, nicotinamide, nicotinamide riboside, nicotinamide mononucleotides, peptides, amino acids, carnosine, exosomes, and the like, but are not necessarily limited thereto. The physiologically active substance may be included in an amount of 0.01% to 3.0% by weight, preferably 0.05% to 2.5% by weight, and more preferably 0.05% to 2.0% by weight based on the percentage by weight of the total composition. When the amount of the physiologically active substance is less than 0.01% by weight, the physiologically active effect due to the addition of the physiologically active substance may be minimal, and when the amount of the physiologically active substance exceeds 3.0% by weight, there is a problem that the hyaluronic acid gel fused with the biodegradable polymer microgranules of the composition may be decomposed by the physiologically active substance, which is not preferable.

The local anesthetic may include lidocaine, mepivacaine, levobupivacaine, and prilocaine, and components such as local anesthetics may be included. The amount of the local anesthetic may be appropriately adjusted by a person skilled in the art, but it may preferably be contained in an amount of 0.1% to 0.5% by weight based on the percentage by weight of the total composition.

### ADVANTAGEOUS EFFECTS

The composition for tissue repair treatment according to the present invention includes a hyaluronic acid gel fused with biodegradable polymer microgranules, and therefore has the effect of simultaneously exhibiting the tissue repair effect by the hyaluronic acid and the biodegradable polymer for a long time. In addition, the biodegradable polymer microgranules included in the composition of the present invention are prepared using an eco-friendly solvent, and therefore are less harmful to a living organism and the environment, and have a spherical or nearly spherical shape with an uneven rough surface and a diameter of 20 µm to 100 µm, and therefore are easy to inject into tissues, and after injection, they are not phagocytosed by macrophages but are well absorbed, thereby effectively inducing collagen production

Moreover, since the hyaluronic acid gel fused with biodegradable polymer microgranules included in the composition of the present invention has a syringe pressure of 40 N or less when measured at an injection speed of 5 to 15 mm/min with a 27G (I.D. 0.210 mm) syringe, there is the effect that the injection of the composition of the present invention including the same into the tissue can be easy and the pain for the person receiving the injection can be reduced. In addition, since the composition of the present invention is present in a gel form, a separate dilution or stirring process is unnecessary before injecting into the tissue, and the problem of heterogeneity occurring in the dilution or stirring process does not occur, and therefore there is the effect that not only can the composition be injected right away, but the pain for the person receiving the injection can be reduced.

### DESCRIPTION OF THE DRAWINGS

FIG. 1 illustrates a method of preparing an injection composition for tissue repair treatment according to one embodiment of the present invention.
FIG. 2 illustrates a state in which an injection composition for tissue repair treatment according to one embodiment of the present invention (Example 3) is packed in a pre-filled syringe.
FIG. 3 illustrates the fine surface structure of an injection composition for tissue repair treatment according to one embodiment of the present invention (Example 3) confirmed and photographed using scanning electron microscope (SEM).
FIG. 4 illustrates the fine surface structure of Comparative Example 3 confirmed and photographed using SEM for comparison with an injection composition for tissue repair treatment according to one embodiment of the present invention.

### BEST MODE

Hereinafter, the present invention will be described in detail. However, these examples are intended to exemplify the present invention, and the scope of the present invention is not limited to these examples.

### <Example 1>

5% (w/w) of poly-L-lactic acid (PLLA) having a weight-average molecular weight of 200,000 g/mol or less was dissolved in dimethyl sulfoxide at room temperature, and the resulting solution was sprayed and frozen at a rate of 5 mL/min at -10 °C, washed with ethanol, and freeze-dried to prepare polymer microgranules. Meanwhile, 10% (w/w) of hyaluronic acid was dissolved by stirring in an alkaline aqueous solution prepared by dissolving 0.04 g of NaOH in 100 g of purified water, and 2% (w/w) of the freeze-dried polymer microgranules and 1% (w/w) of 1,4-butanediol diglycidyl ether (BDDE) were added, stirring was stopped, and maturation was performed at room temperature for 24 hours to prepare a crosslinked gel. The crosslinked gel was dialyzed with 1X phosphate-buffered saline (PBS) buffer for 3 to 14 days until the crosslinking agent disappeared, and 0.3% (w/w) of lidocaine hydrochloride was added to adjust the final hyaluronic acid concentration to 2% (w/w). The final fusion gel was pulverized to 100 µm, packed into a pre-filled syringe, and sterilized.

### <Example 2>

15% (w/w) of PLLA having a weight-average molecular weight 200,000 g/mol or less was added to a betaine/glycerin/water eutectic solvent, and the resulting mixture was autoclaved (160 °C, 0.12 MPa, 20 minutes) and sonicated while lowering the temperature to obtain microgranules, which were washed and freeze-dried. Meanwhile, 10% hyaluronic acid was dissolved by stirring under alkaline conditions where 0.04 g of NaOH was dissolved in 100 g of purified water, and 1% BDDE was added and mixed, and the resulting mixture was maturated for 24 hours at room temperature under nitrogen conditions to prepare a crosslinked gel. The crosslinked gel was dialyzed with 1X PBS buffer for 3 to 14 days until the crosslinking agent disappeared, and the microgranule polymer (2%) and lidocaine hydrochloride (0.3%) were added to hydrate the crosslinked gel and adjust the final hyaluronic acid concentration to 2% (w/w). The polymer-fused hyaluronic acid gel was pulverized to 100 µm, packed into a pre-filled syringe, and sterilized.

### <Example 3>

15% (w/w) of PLLA having a weight average molecular weight of 200,000 g/mol or less was added to triacetin, and then the resulting mixture was autoclaved (170 °C, 0.12 MPa, 20 minutes), and sonicated while gradually lowering the temperature to obtain microgranules, which were then washed and freeze-dried. Thereafter, a hyaluronic acid gel fused with the 2% polymer microgranules was prepared through the same process as Example 2, and then packed into a pre-filled syringe, and sterilized.. The final properties are as shown in FIG. 2.

### <Example 4>

The preparation was performed by the same process as Example 3, except that the concentration of the mixed biodegradable polymer microgranules was 12%.

### <Example 5>

The preparation was performed by the same process as Example 3, except that the concentration of the mixed biodegradable polymer microgranules was 5%.

### <Example 6>

The preparation was performed by the same process as Example 3, except that the concentration of the mixed biodegradable polymer microgranules was 1%.

### <Comparative Example 1>

Commercially available Scultra (a freeze-dried product in which polylactic acid and carboxymethyl cellulose are mixed) was purchased and used.

### <Comparative Example 2>

10% hyaluronic acid was dissolved by stirring under alkaline conditions (pH 12), 1% BDDE was added to the resulting mixture, and then stirring was stopped and the gel was maturated at room temperature for 24 hours to prepare a crosslinked gel. The crosslinked gel was dialyzed with 1X PBS buffer for 3 to 14 days until the crosslinking agent disappeared, and lidocaine hydrochloride (0.3%) was added to adjust the final concentration of hyaluronic acid to 2.4%. Thereafter, the product was pulverized to 100 µm, packed in a pre-filled syringe, and sterilized.

### <Comparative Example 3>

5% (w/w) of PCL having a weight-average molecular weight of 50,000 g/mol or less was dissolved in methylene chloride, a halogenated organic solvent, and the resulting solution was passed through a 30 µm pore Shirasu porous glass (SPG) membrane column to be injected into a 1% polyvinylalcohol (PVA) aqueous solution containing a surfactant, thereby preparing a microemulsion, which was washed and freeze-dried to produce microparticles.

### <Comparative Example 4>

The same process as Comparative Example 3 was performed, except that 5% (w/w) PLLA having a weight average molecular weight of 200,000 g/mol or less was used.

### Experimental Example 1. Confirmation of the particle size of microgranules of biodegradable polymer

The biodegradable polymer microgranules prepared in Examples 1 to 3 were mixed with purified water at a concentration of 0.5%, vortexed for 10 seconds, dispersed by sonication (1 minute), and the dispersion was observed under an optical microscope, and the particle size was measured. In addition, the microparticles prepared in Comparative Examples 3 and 4 were observed using the same method, and the particle size was measured.

**[Table 1]**

| | Example 1 | Example 2 | Example 3 | Comparative Example 3 | Comparative Example 4 |
|---|---|---|---|---|---|
| Size (um) | 20-40 | 40-70 | 50-100 | 20-40 | 20-50 |

As can be seen from Table 1 above, it was confirmed that in Examples 1 to 3 prepared based on an eco-friendly solvent, microparticles were formed, similar to Comparative Examples 3 and 4 prepared using a conventional halogenated organic solvent and a surfactant.

### Experimental Example 2. Confirmation of the surface of biodegradable polymer microgranules

The surface microstructure of the biodegradable polymer microgranules prepared in Example 3 and the surface structure of the microparticles of Comparative Example 3 were confirmed by scanning electron microscopy (SEM) as shown in FIGS. 3 and 4. As a result, it was confirmed that the microgranules of Example 3 had an uneven surface (see FIG. 3), but the surface of Comparative Example 3 was a smooth sphere-type surface (see FIG. 4). When the surface of the biodegradable polymer microgranules of the present invention was modified to be uneven, the dispersion stability in the aqueous hydrogel was increased, and therefore it was easy to form a stable fused filler. In contrast, in the cases where the surface of the particles is smooth, as in Comparative Example 3, when hydrophobic biodegradable polymer particles are dispersed in a hyaluronic acid hydrogel and then subjected to wet sterilization, the gap between the oil and water phases becomes larger, and therefore bubbles are formed easily, which is a problem that the particles become unsuitable for the preparation of a stable fused filler that may be commercialized.

### Experimental Example 3. pH and phase stability of hyaluronic acid filler fused with biodegradable polymer

The pH of the fillers of Examples 1 to 3 and Comparative Examples 1 to 3 prepared above was measured using a pH meter (Thermo scientific, Orion star A211), and the phase stability was observed at one-week intervals after storage at room temperature for two weeks. Since Comparative Examples 1 and 3 were powder phases, the phases were compared by dispersing them in purified water at a concentration of 4% (w/v) corresponding to the combined weight of hyaluronic acid and microgranules of Examples 1 to 3.

**[Table 2]**

| | Exampl e 1 | Exampl e 2 | Exampl e 3 | Compara tive Example 1 | Compara tive Example 2 | Compara tive Example 3 | Compara tive Example 4 |
|---|---|---|---|---|---|---|---|
| Da y 0 | pH 7.29 Turbid gel phase | pH 7.15 Turbid gel phase | pH 6.93 Turbid gel phase | pH 6.51 Precipi tated gel phase | pH 7.23 Clear gel phase | pH 6.70 Precipi tated gel phase | pH 7.19 Precipi tated gel phase |
| We ek 1 | pH 7.46 Phase mainta ined | pH 7.22 Phase mainta ined | pH 7.01 Phase mainta ined | pH 6.50 Precipi tated liquid phase | pH 7.24 Phase maintai ned | pH 6.67 Precipi tated liquid phase | pH 7.14 Precipi tated liquid phase |
| We ek 2 | pH 7.38 Phase mainta ined | pH 7.18 Phase mainta ined | pH 6.95 Phase mainta ined | pH 6.49 Precipi tated liquid phase | pH 7.23 Phase maintai ned | pH 6.65 Precipi tated liquid phase | pH 7.00 Precipi tated liquid phase |

As can be seen from the above Table 2, it was confirmed that the hyaluronic acid fillers (Examples 1 to 3) fused with biodegradable polymers were stable in terms of pH and phase. On the other hand, in the case of Comparative Examples 1, 3, and 4, the microparticles were not in a homogeneous state and but were precipitated in water, which cause inconvenience to the practitioner when used as an injection solution.

### Experimental Example 4. Stability of injection force of hyaluronic acid filler fused with biodegradable polymer

An injection force stability test was conducted with the fillers of Examples 1 to 3 and Comparative Examples 1 to 3 prepared above. It was determined whether the pressure required to inject the contents of the syringe during filler injection was 40 N or less and whether the pressure was uniform, and the results were used for the product specifications (AND, MCT-2150). Comparative Examples 1 and 3 were dispersed in purified water at a concentration of 4% (w/w) equal to the combined weight of hyaluronic acid and microgranules of Examples 1 to 3, and then filled into a pre-filled syringe for comparison. When the injection force (N) was measured at a speed of 10 mm/m using a 1 mL syringe and a 27 G injection needle, the standard deviation of the average injection force for 20 to 60 seconds after injection was expressed as <5%: +++, <15%: ++, and <25%: +.

**[Table 3]**

| | Examp le 1 | Examp le 2 | Examp le 3 | Comparat ive Example 1 | Comparat ive Example 2 | Comparat ive Example 3 | Comparat ive Example 4 |
|---|---|---|---|---|---|---|---|
| Day 1 | +++ | +++ | +++ | ++ | +++ | + | + |
| Wee k 1 | +++ | +++ | +++ | ++ | +++ | + | + |
| Wee k 2 | +++ | +++ | +++ | ++ | +++ | ++ | + |

As can be seen from the above Table 3, the hyaluronic acid fillers (Examples 1, 2, and 3) fused with biodegradable polymers showed stable injection power, indicating that they were homogeneous phases, and it was determined that there would be no problems such as clogging of the injection needle. In addition, it was confirmed that the injection could be readily performed without the need to separately prepare water for injection and suspend the filler during the procedure, and since hyaluronic acid and biodegradable polymer are injected in a single procedure, the formulation may provide convenience for the procedure.

On the other hand, Comparative Examples 1, 3, and 4, excluding Comparative Example 2, did not show stable injection power, indicating that they were heterogeneous phases, and it was expected that problems such as clogging of the injection needle could occur. In addition, Comparative Example 2 was a filler composed only of a general hyaluronic acid gel and exhibited a stable injection force, indicating that it was a homogeneous phase. However, it was confirmed that despite the fact that biodegradable polymer microgranules were fused, the hyaluronic acid filler fused with biodegradable polymer microgranules of the present invention exhibited a stable injection force, similar to the general hyaluronic acid gel filler of Comparative Example 2.

### Experimental Example 5. pH of hyaluronic acid filler fused with biodegradable polymer

The pH of Examples 3 to 6 was measured using the same method as Experimental Example 2.

**[Table 4]**

| | Example 3 | Example 4 | Example 5 | Example 6 | Comparative Example 2 |
|---|---|---|---|---|---|
| pH | 6.93 | 5.50 | 7.01 | 7.15 | 7.23 |

From the results in Table 4, it was confirmed that increasing the proportion of the biodegradable polymer (Example 4) compared to Example 3 increased the acidity and thus was not suitable for injection, and in the case of Comparative Example 2, it was confirmed that the pH was neutral like the existing hyaluronic acid filler because there was no biodegradable polymer.

### Experimental Example 6. Efficacy test of hyaluronic acid filler fused with polymer

After injecting 200 µL of Examples 1 to 3 and Comparative Example 1 into the back of hairless mice, the volume change was measured, and the results are shown in Table 5 below. As a result, Examples 1 to 3, 5, and 6 showed a significant decrease in the initial volume decrease after one month, compared with the volume decrease observed when treating with a simple mixture formulation of polylactic acid and carboxymethyl cellulose (Comparative Example 1), and exhibited a continuous volume maintenance effect after 3 months, in contrast to Comparative Example 1.

**[Table 5]**

| | Exampl e 1 | Exampl e 2 | Exampl e 3 | Exampl e 5 | Exampl e 6 | Comparativ e Example 1 |
|---|---|---|---|---|---|---|
| Volume immediat e after procedur e | 100% | 100% | 100% | 100% | 100% | 100% |
| Volume after one month | 80% | 86% | 80% | 85% | 90% | 30% |
| Volume after three months | 75% | 79% | 78% | 80% | 87% | 35% |

## Claims

1. A method of preparing an injection composition for tissue repair treatment including a hyaluronic acid gel fused with biodegradable polymer microgranules prepared using an eco-friendly solvent, the method comprising:
(S10) a step of preparing microgranules of a biodegradable polymer using an eco-friendly solvent;
(S20) a step of preparing a hyaluronic acid gel; and
(S30) a step of dispersing the biodegradable polymer microgranules of Step (S10) in the hyaluronic acid gel of Step (S20),
wherein the step (S10) includes: (A10) a step of stirring and dissolving the biodegradable polymer in an eco-friendly solvent to prepare a biodegradable polymer solution; (A20) a step of spraying and freezing the biodegradable polymer solution of Step (A10) at a low temperature; and (A30) a step of removing the solvent from the frozen biodegradable polymer solution of Step (A20); or
(B10) a step of mixing a biodegradable polymer and an eco-friendly solvent and dissolving the biodegradable polymer at a high temperature and a high pressure to prepare a biodegradable polymer solution; (B20) a step of sonicating a resulting melt of the biodegradable polymer of Step (B10); and (B30) a step of removing the solvent from the melt of Step (B20).

2. The method according to claim 1, wherein the biodegradable polymer is one or more selected from the group consisting of poly-L-lactic acid, poly-D-lactic acid, poly-DL-lactic acid, polylactic acid, polyglycolic acid, polydioxanone, polycaprolactone, poly-α-hydroxy acid, poly(trimethylene carbonate), poly(alkylcyanoacrylate), polyhydroxyalkanoate, methoxypolyethylene glycol, hydroxyl polyethylene glycol, monoalkoxy polyethylene glycol, polyethylene glycol, a copolymer thereof, and a mixture thereof.

3. The method according to claim 1, wherein the eco-friendly solvent is one or more organic solvents selected from the group consisting of dimethyl sulfoxide, acetic acid, acetone, anisole, 1-butanol, 2-butanol, butyl acetate, tert-butyl methyl ether, ethanol, ethyl acetate, ethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, dihydrolevoglucosenone, methyl-5-(dimethylamino)-2-methyl-5-oxopentanoate, triethyl phosphate, ethylene carbonate, propylene carbonate, butylene carbonate, glycerol carbonate, diethyl carbonate, dimethyl carbonate, methyl lactate, ethyl lactate, tetraglycol, and a mixture thereof, triacetin, or a eutectic solvent.

4. The method according to claim 1, wherein the eutectic solvent is a mixture of one or more hydrogen bond acceptors selected from the group consisting of choline chloride, betaine, sodium acetate, proline, nicotinamide, L-carnitine, and combinations thereof and one or more hydrogen bond donors selected from the group consisting of polyols, organic acids, fatty acids, amide compounds, sugars, and combinations thereof.

5. The method according to claim 1, wherein the biodegradable polymer microgranules have a spherical or nearly spherical shape with a rough surface and a diameter of 20 to 100 µm.

6. The method according to claim 1, wherein in Step (A10) or Step (B10), an eco-friendly solvent is mixed in an amount of 200 to 10,000 parts by weight based on 100 parts by weight of the biodegradable polymer.

7. The method according to claim 1, wherein the low temperature of Step (A20) is -25 to -5 °C.

8. The method according to claim 1, wherein the dispersion in Step (S30) consists of hyaluronic acid and the biodegradable polymer microgranules in a weight ratio of 1:0.5 to 5.

9. The method according to claim 1, wherein the dispersion in Step (S30) further includes one or more selected from the group consisting of purified water, a physiologically active substance, and a local anesthetic, in addition to the hyaluronic acid gel and the biodegradable polymer microgranules.

10. An injection composition for tissue repair treatment, comprising a hyaluronic acid gel fused with microgranules of a biodegradable polymer prepared using an eco-friendly solvent,
wherein the hyaluronic acid is included in an amount of 1% to 5% by weight based on the total weight of the composition, and the microgranules of the biodegradable polymer are included in an amount of 1% to 10% by weight based on the total amount,
wherein the microgranules of the biodegradable polymer are prepared by a method including: (A10) a step of stirring and dissolving the biodegradable polymer in an eco-friendly solvent to prepare a biodegradable polymer solution; (A20) a step of spraying and freezing the biodegradable polymer solution of Step (A10) at a low temperature; and (A30) a step of removing the solvent from the frozen biodegradable polymer solution of Step (A20); or (B10) a step of mixing a biodegradable polymer and an eco-friendly solvent and dissolving the biodegradable polymer at a high temperature and a high pressure to prepare a biodegradable polymer solution; (B20) a step of sonicating a resulting melt of the biodegradable polymer of Step (B10); and (B30) a step of removing the solvent from the melt of Step (B20).

11. The injection composition for tissue repair treatment according to claim 10, wherein the biodegradable polymer microgranules have a spherical or nearly spherical shape with a rough surface and a diameter of 20 to 100 µm.

12. The injection composition for tissue repair treatment according to claim 10, wherein the hyaluronic acid and the biodegradable polymer microgranules are included in a weight ratio of 1:0.5 to 5.

13. The injection composition for tissue repair treatment according to claim 10, wherein the biodegradable polymer is one or more selected from the group consisting of poly-L-lactic acid, poly-D-lactic acid, poly-DL-lactic acid, polylactic acid, polyglycolic acid, polydioxanone, polycaprolactone, poly-α-hydroxy acid, poly(trimethylene carbonate), poly(alkylcyanoacrylate), polyhydroxyalkanoate, methoxypolyethylene glycol, hydroxyl polyethylene glycol, monoalkoxy polyethylene glycol, polyethylene glycol, a copolymer thereof, and a mixture thereof.

14. The injection composition for tissue repair treatment according to claim 10, wherein the eco-friendly solvent is one or more organic solvents selected from the group consisting of dimethyl sulfoxide, acetic acid, acetone, anisole, 1-butanol, 2-butanol, butyl acetate, tert-butyl methyl ether, ethanol, ethyl acetate, ethyl ether, ethyl formate, formic acid, heptane, isobutyl acetate, isopropyl acetate, methyl acetate, 3-methyl-1-butanol, methyl ethyl ketone, methyl isobutyl ketone, 2-methyl-1-propanol, pentane, 1-pentanol, 1-propanol, 2-propanol, propyl acetate, dihydrolevoglucosenone, methyl-5-(dimethylamino)-2-methyl-5-oxopentanoate, triethyl phosphate, ethylene carbonate, propylene carbonate, butylene carbonate, glycerol carbonate, diethyl carbonate, dimethyl carbonate, methyl lactate, ethyl lactate, tetraglycol, and a mixture thereof, triacetin, or a eutectic solvent.

15. The injection composition for tissue repair treatment according to claim 14, wherein the eutectic solvent is a mixture of one or more hydrogen bond acceptors selected from the group consisting of choline chloride, betaine, sodium acetate, proline, nicotinamide, L-carnitine, and combinations thereof and one or more hydrogen bond donors selected from the group consisting of polyols, organic acids, fatty acids, amide compounds, sugars, and combinations thereof.

16. The injection composition for tissue repair treatment according to claim 10, wherein the dispersion Step (S30) further includes one or more selected from the group consisting of purified water, a physiologically active substance, and a local anesthetic, in addition to the hyaluronic acid gel and the biodegradable polymer microgranules.
